# EUROPEAN PATENT SPECIFICATION

(11) **EP 2 025 620 B1**
(45) Date of publication and mention of the grant of the patent: **01.02.2017**
(21) Application number: 07730482.2
(22) Date of filing: 27.02.2007
(51) Int. Cl.: C12N 1/36, B65D 81/28, A61K 36/53, A61K 36/54, A61K 36/61

(54) **ACTIVE PACKAGING THAT INHIBITS FOOD PATHOGENS**
LEBENSMITTELPATHOGENE HEMMENDE AKTIVE VERPACKUNG
EMBALLAGE ACTIF INHIBITEUR DE PATOGÈNES ALIMENTAIRES

(30) Priority: 08.06.2006 ES 200601550
(43) Date of publication of application: 18.02.2009
(73) Proprietor: Repsol Lubricantes y Especialidades, S.A., 28045 Madrid (ES)
(72) Inventor: NERÍN DE LA PUERTA, Cristina, 28043 Madrid (ES); ASTUDILLO CAMPILLO, Marisa, 28043 Madrid (ES); COVIÁN SÁNCHEZ, Ignacio, 28043 Madrid (ES); MUJIKA GARAI, Ramón, 28043 Madrid (ES)
(74) Representative: Pons
(86) International application number: PCT/ES2007/070039
(87) International publication number: WO 2007/144444

(56) References cited:
- EP-A1- 0 842 099
- EP-A1- 1 657 181
- WO-A1-01/49121
- WO-A1-98/36993
- JP-A- 2005 219 759
- US-B1- 6 482 452
- LOPEZ P ET AL: "Solid- and vapor-phase antimicrobial activities of six essential oils: Susceptibility of selected foodborne bacterial and fungal strains" JOURNAL OF AGRICULTURAL AND FOOD CHEMISTRY, vol. 53, no. 17, August 2005 (2005-08), pages 6939-6946, XP002592931 ISSN: 0021-8561
- RODRIGUEZ ET AL: "The use of natural essential oils as antimicrobial solutions in paper packaging. Part II" PROGRESS IN ORGANIC COATINGS, ELSEVIER BV, NL LNKD- DOI:10.1016/J.PORGCOAT.2007.06.006, vol. 60, no. 1, 24 August 2007 (2007-08-24), pages 33-38, XP022210640 ISSN: 0300-9440

## Description

This invention relates to a new active food pathogen inhibitor packaging, either by the generation of an active atmosphere or by direct contact, which comprises a paper, cardboard, cork, aluminum or wood support and an active coating thereof. Said coating consists of a formulation of paraffin and natural plant extracts.

### PRIOR ART

The loss of quality of packaged foods is produced by different causes, which include microorganisms (bacteria, fungi [yeasts and mildews]), temperature, sunlight, oxygen or humidity. These factors cause microbial proliferation in the foods, changes in the vitamins, alterations in the organoleptic properties and oxidation of fats (rancid taste), with the consequent rejection.

As a result, there are many products that act as anti-microbial agents and are used to protect foods, amongst them, certain natural plant extracts. Patent US 20040213861 discloses a method and a composition designed to inhibit the growth of microorganisms in food products, which consists of using thyme and cinnamon as anti-microbial agents. Several components of cinnamon oil are known (Lopez P et al. 2005 J Agric Food Chem 53(17):6939-6946).

On the other hand, the need to transport and store foods has led to the development of packagings and/or wrappings that protect these foods against contamination by microbial infections. For example, International Patent WO 01/49121 discloses a packaging designed to preserve meat products which contains an anti-microbial agent. Spanish Patent ES 2144761 also discloses a packaging designed to preserve edible products which contains a preservative agent (rosemary or thyme oil, or a combination thereof). Likewise, patent US 6482452 discloses a waterproof packaging for meat that incorporates plant-based preservatives. These packagings act by direct contact with the foods. Other packagings that do not need direct contact with the food have been described, for example active packagings for food using natural products such as cinnamon, clove, ginger, rosemary, oregano, dill or basil (EP1657181A1).

The use of paraffins as an anti-humidity barrier is also well-known in the state of the art. This material is cited, amongst others, in patent GB 669942, which discloses a wrapping that contains a layer of paraffin wax with para nitro benzoic acid, ethyl paranitrobenzoate, which is a non-volatile compound that requires direct contact with the product, designed to protect foods against mildew up to 12 days. In JP2005219759A paraffin is also used in an active packaging for foods capable of inhibiting the growth of food pathogens. Moreover, patent GB 473318 discloses the use of paraffin with chloramine T (as germicidal gases), which is an inorganic and toxic compound, to preserve perishable foods and to prevent moisture, and patent US 2480010 discloses a packaging designed to preserve organic matter against the development of fungi, which contains a coating made of paraffin waxes and dichlorosuccinic acids or the esters thereof which is incorporated in the paraffin by a hot-melt technique, wherein the preservation is maintained up to 25 days of exposure.

### EXPLANATION OF THE INVENTION

The authors of this invention have developed an alternative active packaging system to be used in the food industry as a packaging for foods that is capable of inhibiting the growth of food pathogens due to the generation of an active atmosphere or direct contact with the food.

Paraffin by itself is not only used as an anti-humidity barrier in food packaging, but also as a carrier of pathogen inhibitor agents to protect foods against deterioration by fungi, or bacteria.

The packaging may play an important role in preventing food deterioration. The positive migration of antioxidant, anti-microbial or aromatic substances from inside the packaging to the food is what creates the active packaging. An active packaging is defined as that which supplies compounds that protect the food or absorbs those that deteriorate it.

In a first aspect of the invention, an active packaging for foods is disclosed that is capable of inhibiting the growth of food pathogens, either by generating an active atmosphere or by direct contact, which comprises a support and an active coating of said support that is composed of paraffin and cinnamon essential oil fortified and additived with cinnamaldehyde, wherein the fortified cinnamon is incorporated into the paraffin.

The term "active packaging" in this invention refers to an active system which comprises a material that acts as a support and a coating made of a paraffin-based or additived paraffin formulation that incorporates active agents. This ensemble behaves as an active packaging system.

Paraffins are used to form a moisture-repellent barrier and protect foods against humidity. This moisture-repellent barrier is prepared by depositing a thin paraffin layer on the surface of the support material that is to be in contact with the food, taking advantage of their highly hydrophobic properties. In addition to their great versatility, ease of handling and low price, paraffins are not very reactive, for which reason they do not alter the foods' qualities. These compounds are specially indicated for the coating of paper in packagings and of cardboard or wood boxes designed for the transport of fruits, vegetables, meat, fish, dairy products and foods in general, whether moist, greasy or dry. They are also suitable for coating any other type of support that will be in contact with this type of foods.

Likewise, any coating material with physicochemical properties similar to those of paraffin may be used.

The paraffins or related materials that are considered to be most widely used, but not limited thereto, are the following:
1. Macrocrystalline (hydrogenated) paraffins CAS: 64742-51-4
2. Microcrystalline (hydrogenated) paraffins CAS: 92045-76-6
3. Synthetic paraffins (including Fisher Trop paraffins):
   - Polyethylene CAS: 099002-88-4
   - Oxidised polyethylene CAS: 068441-17-8
   - Polypropylene waxes CAS: 009003-07-0
4. White oils CAS: 801295-1
5. Natural waxes or esters of vegetable or animal origin:
   - Carnauba wax CAS: 008015-86-9
   - Candelilla wax CAS: 008006-44-8
   - Bee wax CAS: 008012-89-3
   - Japanese wax CAS 008001-39-6
   - Montanic waxes CAS: 008002-53-7
   - Stearic oil CAS: 000057-11-4.

An essential requirement to use these products or such like in this invention is that they must be suitable for use in contact with foods. This is normally achieved by hydrotreatment or an equivalent process and must comply with the different prevailing food regulations, such as FDA, European Union, Mercosur or the regulations in force in the country of use.

Table 1 indicates the main paraffins of petroleum origin susceptible of being used as carriers of these active agents, provided that they comply with the above-mentioned condition.

**Table 1. Petroleum paraffins susceptible of being used as active agent carriers.**

| Description | CAS No. |
|---|---|
| Paraffin waxes and hydrocarbon waxes | 8002-74-2 |
| Paraffin waxes and microcrystalline hydrocarbon waxes | 63231-60-7 |
| Paraffin waxes (petroleum), treated with acid | 64742-26-3 |
| Paraffin waxes (petroleum), chemically neutralised | 64742-33-2 |
| Paraffin waxes (petroleum), microcrystalline, treated with earths | 64742-42-3 |
| Paraffin waxes (petroleum), treated with earths | 64742-43-4 |
| Paraffin waxes (petroleum), hydrotreated | 64742-51-4 |
| Hydrocarbon waxes (petroleum), microcrystalline, hydrotreated | 64742-60-5 |
| Hydrocarbon waxes (petroleum), deodorised | 85029-72-7 |
| Paraffin waxes (petroleum), treated with acid | 90669-47-9 |
| Paraffin waxes (petroleum), low molecular weight | 92045-74-4 |
| Paraffin waxes (petroleum), low molecular weight, hydrotreated | 92045-75-5 |
| Paraffin waxes and hydrocarbon waxes, micro., hydrotreated | 92045-76-6 |
| Paraffin waxes and hydrocarbon waxes C19-38 | 97489-05-9 |
| Paraffin waxes (petroleum), treated with active carbon | 97862-89-0 |
| Paraffin waxes (petroleum), low molecular weight, /active carbon | 97862-90-3 |
| Paraffin waxes (petroleum), low molecular weight with earths | 97862-91-4 |
| Paraffin waxes (petroleum), low molecular weight, /silicic acid | 927862-92-5 |
| Paraffin waxes (petroleum), treated with silicic acid | 97862-93-6 |
| Paraffin waxes and hydrocarbon waxes, micro. /active carbon | 97862-94-7 |
| Paraffin waxes and hydrocarbon waxes, micro., /earth | 97862-95-8 |
| Paraffin waxes and hydrocarbon waxes, micro., /silicic acid | 97862-96-9 |
| White oil | 801295-1 |

Natural essence extracts are from cinnamon, clove, oregano or thyme, with percentages of incorporation into paraffin that vary between 0.5% and 15% of the total weight, preferably between 1% and 10%, and more preferably between 2% and 6%. Specifically, said active packaging contains cinnamon II extract (cinnamon essential oil fortified and additived with cinnamaldehyde), which is the natural extract with the greatest inhibitory potential against the growth of microorganisms, partially inhibiting the growth of Gram- bacteria and totally inhibiting mildews and yeast at a percentage of incorporation into the active packaging of 1%-6% by weight.

An active packaging is coated with at least cinnamon, thyme, clove or oregano extract, or any combination thereof.

Essential oils are liquids that contain relatively volatile compounds which are generally insoluble in water, but soluble in organic-type solvents. Most are not oily to the touch and have a penetrating odour. Depending on their chemical structure, the components may be grouped in up to five different chemical families: alcohols, esters, ketones, aldehydes and acids. Essential oils come from different parts of vegetables: roots, stem, bark, leaves, flowers or fruits.

The most common method of obtaining essential oil is by carrier vapour distillation, although it is also obtained by extraction, either with solvents or with supercritical fluids, and subsequently it is usually subject to a purification process by vacuum distillation.

The main components of essential oils are aliphatic and aromatic hydrocarbons, alcohols, aldehydes, esters and ketones. From the standpoint of active paraffin, the most important, albeit minority, components are the biological derivatives of mevalonic acid, classified as terpenes. Terpene derivatives (C10 monoterpenes and C15 sesquiterpenes) may be the main agents responsible for the anti-microbial properties of essential oils.

As active agents, extracts of natural cinnamon, clove, oregano or thyme essences have been used. In the assays performed with active paraffin, the best results were obtained with essential oils of natural cinnamon (Cinnamon = Cinnamon zeylanicum CAS No.: 80591-6), called Cinnamon I, and additived cinnamon, which we will call Cinnamon II. The essential oil of cinnamon II is a derivative of the essential oil of cinnamon fortified with the terpene derivative cinnamaldehyde, which is presumed to be one of the main agents responsible for the anti-microbial action of cinnamon essence, and with other components that act as fixation agents for volatile compounds. In addition to those mentioned, other components of the essential oils of cinnamon are, for example, alpha-pinene, estragol or beta-caryophyllene.

The exact composition of the essential oil of cinnamon is unknown, as it varies widely depending on various factors: species of cinnamon used, part of the plant wherefrom the essential oil has been obtained, etc.

In the examples of this invention, it has been shown that active paraffin using the formulation called cinnamon (II) has a clear anti-microbial and antifungal effect, with total inhibition in mildews and yeasts at a concentration equal to or greater than 2% by weight of essence formulated with respect to paraffin.

It is also observed that, once the active paraffin is prepared, it preserves activity for at least 70 days prior to being used when stored at ambient temperature.

As mentioned above, the active packaging of this invention may be composed of any compound capable of being used to package foods and capable of supplying the active agents disclosed in this invention, such that they protect the food or absorb those agents that deteriorate it. In a preferred embodiment of the invention, said compound is a formulation of solid paraffin, additived or not, or a paraffin emulsion. By paraffin emulsion we mean a dispersion of paraffin particles in water, together with additives with an approximate particle size of one micron and a minimum stability of 3 months. In an even more preferred embodiment of the invention, an emulsion called REDEMUL P-58 is used which, in addition to paraffin, incorporates other components in its formulation that are capable of stabilising the sample and, on the other hand, gradually releasing the active agents incorporated. This emulsion is an anionic/non-ionic emulsion with a 50% solid content, a great coating capacity and highly resistant to hard waters. In another even more preferred embodiment, an additived solid paraffin called REDECOAT P-400 is used, which, in addition to paraffin, incorporates other components in its formulation that are capable of stabilising the sample and, on the other hand, gradually releasing the active agents incorporated.

Moreover, the paraffins or similar products must comply with a number of physicochemical characteristics in order to be used in the field of this invention, depending on whether they are solid or emulsion paraffins.

The characteristics of solid paraffin may be determined by means of several equivalent standards; Table 2 specifies the ranges that these products must meet.

**Table 2. Physicochemical characteristics typical of paraffins**

| Physicochemical characteristic | Measurement method | Range of values |
|---|---|---|
| Melting point (°C) | ASTM D-127 | 15-120 |
| Kinematic viscosity (cSt)* | ASTM D-445 | 2_{100°C}-10,000_{130°C} |
| Penetration (dmm) | ASTM D-1321 | <25 |
| Saybolt colour | ASTM D-156 | (+30)-(-10) |
| Oil content (p%) | ASTM D-721 | <3 |
| Hydrocarbon distribution | Gas chromatography | C12-C90 |

| | | |
|---|---|---|
| *ASTM: American Society for Testing and Materials (cSt) centistokes* * *The viscosity of these products has a very broad range. Moreover, it may be measured at several temperatures (the subscript indicates the measurement temperature), which broadens the range of values even more. The viscosity may also be measured as dynamic viscosity.* | | |

In general there are no standardised methods to determine the characteristics of emulsion paraffins; for this reason, they must be determined using internal methods. Table 3 specifies the ranges that emulsions must comply with in order to act as anti-humidity barriers and as active packagings.

**Table 3. Physicochemical characteristics typical of emulsions**

| Physicochemical characteristic | Measurement method | Range of values |
|---|---|---|
| Solid content (%) | Evaporation | 15-70 |
| Viscosity (cPs)* | Rotational viscosimeter | 2_{25°C}-10,000_{25°C} |
| pH | Ag/AgCl electrode | 1-12 |
| Mechanical stability | Pumping | STABLE |

| | | |
|---|---|---|
| *(cPs) centipoises* * *The viscosity of these products has a very broad range. Moreover, it may be measured at several temperatures (the subscript indicates the measurement temperature), which broadens the range of values even more, but it is habitually measured at ambient temperature.* | | |

Paraffin may be hot-applied on the support, using melted solid paraffin, or cold-applied, using emulsion paraffin. In order for the paraffin emulsion to be manageable under cold conditions, surfactants may be added so that it is stable over time and has an suitable viscosity at ambient temperature.

Therefore, the paraffin composition of the active packaging of the following invention may incorporate a surfactant that allows it to remain stable over time and have an suitable viscosity at ambient temperature.

The surfactants may be ionic, if they provide an electric charge to the disperse paraffin particles, or non-ionic, if they do not supply an electric charge. Ionic surfactants may, in turn, be anionic, if the charge is negative, or cationic, if the charge is positive. The most common emulsions for this application are anionic or anionic/non-ionic ones, although it is also possible to use cationic or non-ionic emulsions depending on the type of support. In addition to surfactants, other types of additives may be added to the emulsion, such as thickeners, thinners, colouring agents, etc., depending on the specific application wherein the emulsion is to be used.

A great variety of additives may be added to active paraffin in order to achieve greater control over the release of the essential oil components. These fixation agents, added in the suitable proportion, allow for the active components to become incorporated into the packaging atmosphere or the product itself more slowly, that is, progressively.

The paraffins used in the examples of this invention are formulations that contain surfactants or agents that fixate the volatile compounds supplied by the essential oils.

The support of the active packaging disclosed in this invention may be, without it being limited thereto, paper, cardboard, wood, cork or aluminium, and the active coating is added to said support with a preferred grammage of between 0.5 and 30 g/m², more preferably, between 1 and 15 g/m².

The microorganisms whereon the active paraffin has been assayed are representative of those that habitually act as food pathogens: Gram-positive and Gram-negative bacteria, mildews and yeasts. Gram+ bacteria act on meat products in general, sausages and cereals. They may also appear due to bad hygienic practises and water pollution. Gram- bacteria appear in fresh products: meat, poultry, eggs, etc. Moreover, they also arise due to bad food handling of or by fecal contamination. Mildews grow mainly on dry foods, bread, cheese, cereals, dried fruits and also in fruits. The C. *albicans* yeast usually grows on pasta, bread or potatoes. Other yeasts also arise in different fruits varieties.

Some of the Gram-negative bacteria may be P. *aeruginosa,* S. *choleraesuis,* Y. *enterocolitica* or E. *coli.* As Gram-positive bacteria: E. *faecalis,* L. *monocytogenes,* S. *aureus* or B. *cereus.* As yeasts: C. *albicans* or Z. *roxii;* and as mildews: P. *nalgiovense,* E. *repens,* P. *islandicum,* P. *roqueforti,* A. *flavus,* B. *cinerea,* P. *expansum,* P. *digitatum* or D. *hansenii.*

As shown by the examples of this invention, the assays have been performed with cultures of the above-mentioned strains in concentrations that are much higher than those that may be initiated or generated in spontaneous food deterioration. This means that, under normal usage conditions, some of the assayed formulations may also show inhibitory activity, since microorganisms that are not considered to be inhibited in the laboratory assay, because there is not a total reduction in the colony concentration, are altered when they are in high concentrations and, presumably, will be inhibited at low concentrations. Consequently, none of the essences assayed may be discarded.
Therefore, the active packaging of this invention is capable of inhibiting the growth of at least the following food pathogens: Gram-negative bacteria (Escherichia *coli,* Pseudomonas *aeruginosa,* Yersinia *enterocolitica* or Salmonella *choleraesuis),* Gram-positive bacteria (Staphylococcus *aureus,* Lysteria *monocytogenes,* Enterococcus *faecalis* or Bacillus *cereus),* mildews (Penicillium *islandicum,* Penicillium *roqueforti,* Penicillium *nalgiovense,* Aspergillus *flavus* and Eurotium *repens)* or the C. *albicans* yeast.

A second aspect of this invention provides the use of the active packaging for the inhibition of the growth of food pathogens. In particular, its use as an antioxidant, preservative, anti-microbial or antifungal agent in any type of food, dry or moist.

More particularly, the use of the active packaging defined above is described for the inhibition of Gram-negative bacteria (Escherichia *coli,* Pseudomonas *aeruginosa,* Yersinia *enterocolitica* or Salmonella *choleraesuis),* Gram-positive bacteria (Staphylococcus *aureus,* Lysteria *monocytogenes,* Enterococcus *faecalis* or Bacillus *cereus*), mildews (Penicillium *islandicum,* Penicillium *roqueforti,* Penicillium *nalgiovense,* Aspergillus *flavus* or Eurotium *repens)* or the C. *albicans* yeast.

Another aspect of this invention provides the use of the active packaging described above to generate an active atmosphere or by direct contact with the food.

An additional aspect of this invention discloses the method of obtaining the active packaging described above, which comprises the following steps:
a) Preparation of the active paraffin
b) Coating of the support with the paraffin prepared in step a).

In order to prepare the active paraffin, depending on whether it is solid paraffin or emulsion paraffin, the following steps are followed:
In the case of solid paraffin:
   a) the paraffin is melted
   b) the necessary additives are added in the suitable order, temperature and quantity
   c) the cinnamon essential oil fortified and additived with cinnamaldehyde is added
   d) it is stirred and allowed to cool to ambient temperature.

In the case of emulsion paraffin:
e) steps a) to c) for solid paraffin are followed
f) the necessary surfactants are added in the suitable order, temperature and quantity. Those soluble in water are treated in water and, subsequently, this aqueous phase is made to react with the paraffin phase
g) it is passed through a high-pressure homogeniser in order to adjust the particle size
h) it is passed through a heat exchanger in order to leave the emulsion at ambient temperature.

The cinnamon essential oil fortified and additived with cinnamaldehyde is incorporated in a percentage of, preferably, between 2%-10% by paraffin weight. The result is a cream-coloured paraffin with cinnamon odour, the greater the higher the percentage of essential oil incorporated.

In the event that the essential oil added is from cinnamon I or from cinnamon II, it has been shown that, if the percentage of essential oil added to the paraffin formulation is greater than 10%, when the mixture is stirred the paraffin formulation is denatured, and loses its mechanical and physical properties; therefore, it cannot be used for the coating. This behaviour may be extended to other essential oils.

In order to achieve a good anti-humidity barrier, even in cartons that remain in refrigerators for long periods of time, the recommended grammage must be between 1 and 15 g/m², more preferably between 5 and 10 g/m², although selection of the grammage is a function of the paper quality. Low-quality, recycled or low-grammage papers require a greater quantity of paraffin than Kraft-type high-quality, high-grammage papers. The greater the quality of the paper, the longer the duration of the barrier.

The assays performed with different grammages do not show significant differences in anti-microbial behaviour, which means that the main action mechanism takes place from the paraffin surface, wherefrom a protective active atmosphere is generated, with variable concentrations of the volatile components supplied by the anti-microbial agents used (cinnamon (II), etc.).

The emulsion may be applied pure or diluted. The emulsion dilution primarily depends on the speed of the conveyor belt and the temperature of the hot rollers. Although it is advisable to individually adjust the conditions in each machine in order to achieve the suitable grammage, the following table specifies the approximate dilution recommended as a function of the processing conditions:

| Roller temperature (°C) | Belt speed (m/min) | Dilution (% emulsion in water) |
|---|---|---|
| 0-150 | 0-150 | 75% |
| 0-150 | >150 | Pure |
| >150 | 0-150 | 50%/75% |
| >150 | >150 | 75%/Pure |

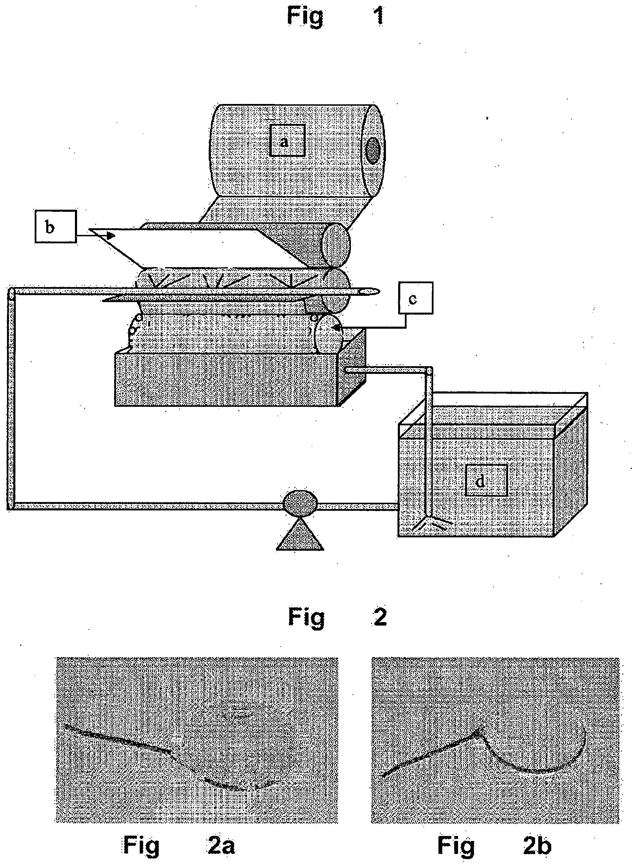

Another aspect of this invention lies in the application of the emulsion. The preferred site of application of the emulsion is on the conveyor belt just before it comes into contact with the hot roller. Coating of the paper by the emulsion is usually performed by means of a system of applicator rollers (see Figure 1).

The process is performed as follows: a unilox-type impregnation roller enters an emulsion bath located just beneath it and bathes the rubber applicator roller, which distributes the emulsion on the paper that passes through the upper part of the roller. The excess emulsion is removed by a blade located at the exit of the applicator device and incorporated into the original reservoir. Dosing of the emulsion on the paper is adjusted with another metal roller located at a controllable distance from the impregnation roller (Figure 1).

As described above, the emulsion dilution primarily depends on the speed of the conveyor belt-and the temperature of the hot rollers, which can be above 150°C. In principle, this may create a problem when applying the new active paraffin, since the essential oil components, which are responsible for the anti-microbial effect, are volatile and come off the support with temperature; consequently, the anti-microbial capacity may decrease depending on the temperature (and duration of the process) whereto they are subject. However, both the paraffin formulation composition and the final mixture contain polar surfactant components, amongst others, capable of fixating the volatile components in the formulation and, therefore, retaining them when the temperature increases, thereby maintaining the final formulation's anti-microbial capacity.

The action mechanism of the new active paraffin follows the same laws that govern processes such as the migration of substances from the packaging to the foods. In this case, the essential oil components, mostly derived from terpene, which are those with anti-microbial capacity, migrate from the surface of the active paraffin to the atmosphere or directly remain on said surface, forming an interface with the food (in the case of direct contact) by a mass transfer mechanism. Moreover, there must also be a mass transfer mechanism whereby the components diffuse from the matrix of the paraffin to the surface thereof. These mechanisms act as long as the corresponding concentration gradients are maintained. However, the results obtained suggest that equilibrium is always achieved, such that, once the compounds are released on the surface, the active paraffin layer is not exhausted, being able to once again regenerate the anti-microbial atmosphere and continue acting for a long time. The independence of anti-microbial activity with respect to the thickness of paraffin applied confirms this behaviour.

In general, the speed at which the material is transferred depends on the nature of the chemical substances transferred, the type of additives used in the active paraffin formulation, the food and also the nature and shape of the packaging.

In this invention, bactericidal effect is understood to mean that which completely annihilates the microorganism, whereas bacteriostatic effect is that which only delays the growth of the microorganism.

In this invention, all the technical and scientific terms have the same meaning as that commonly understood by a person skilled in the art whereto the invention pertains. Throughout the description and the claims, the word "comprises" and the variants thereof are not intended to exclude other technical characteristics, components or steps. For those skilled in the art, other objects, advantages and characteristics of the invention will arise partly from the description and partly from the practise of the invention. The following examples and figures are provided for illustrative purposes and are not intended to limit this invention.

### DESCRIPTION OF THE FIGURES

**Figure 1****.**
   Figure 1 shows the process of coating the paper with the emulsion, where a unilox-type impregnation roller (c) enters into an emulsion bath located right beneath it and bathes the rubber applicator roller, which distributes the emulsion on the paper that passes through the upper part of the roller. The excess emulsion is removed with a blade located at the exit of the applicator device and incorporated into the original reservoir. Dosing of the emulsion on the paper is adjusted with another metal roller, located at a controllable distance from the impregnation roller. The most characteristic parts of Figure 1 are described below.
   a) Virgin paper
   b) Paraffined paper
   c) Impregnation roller
   d) Emulsion container
**Figure 2**
   Figure 2a. Petri dish with active paper (front view)
   Figure 2b. Petri dish with active paper (rear view).
**Figure 3**
   C. *albicans* with 4% cinnamon II essence. Total inhibition is observed.
   This figure shows a control dish (right) and a C. *albicans* dish with 4% cinnamon II extract. One can observe that no growth has taken place in the dish with the paraffined paper (total inhibition).
**Figure 4**
   Dish of A. *flavus* mildew with 4% cinnamon II essence in paraffin (right) and control dish.
   One can observe that no growth has taken place in the dish with the paraffined paper (total inhibition).
**Figure 5**
   Dish of B. *cereus* bacteria with 4% cinnamon II essence in paraffin (right) and control dish.
   The same growth is observed in both dishes; there is no inhibition.
**Figure 6**
   Dish of E. *coli* with 6% cinnamon II (right) and control dish.
   A smaller size of the CFU is observed with respect to those of the control dish.
**Figure 7**
   Partial results of the assay of the anti-microbial paraffin packaging with strawberries.

The image shows the strawberries 6 days after the beginning of the assay. The left of the image shows the packaging with 4% cinnamon II, the centre shows the control packaging, that is, in the paraffined paper WITHOUT cinnamon II essence, and the right of the image shows the packaging with 2% cinnamon II.

Alterations (appearance of mildews, contraction of the fruit piece or exudation of juices) are observed in the strawberries on the control packaging and those on the packaging with 2% cinnamon II. In the packaging with 4% active principle (cinnamon II), we can observe the absence of juice exudation, which is characteristic of the rotting of strawberries, the absence of contraction in the fruit piece and non-proliferation of mildews.

### DETAILED DESCRIPTION OF THE EMBODIMENTS

Below we will illustrate the invention with some assays performed by the inventors, which show the effectiveness of the active paraffin packaging.

### Example 1

In order to obtain the active paraffin packaging: the paraffin prepared as described in the specification of this invention is homogeneously mixed with the natural essential oil in the desired percentage; for example, 6% cinnamon by weight. Subsequently, a sheet of paper is coated with the resulting product; to this end, an automatic coating machine is used, which leads to a more homogeneous coating. Grammage of the coating (3-4 g/m²) is controlled by weighting. Thus, the active paraffined sheets of paper are obtained, which are stored at ambient temperature until the following day, when they are used. Once the active paraffin is prepared, it is applied in the same manner as the conventional paraffin formulation, without active agents.

### Example 2

### 2.1. BASE PARAFFIN USED IN THE EXPERIMENTS

### Solid paraffin:

In order to study the effects of the pathogen inhibitors, a formulation was made with hydrogenated paraffin with CAS No.: 64742-51-4. This preparation complied with the different regulations that affect products in contact with foods (mainly FDA, FDA sections 176.170 and FDA 176.180 and BGVV XXV A). Table 4 specifies the main characteristics of this formulation.

**Table 4. Physicochemical characteristics typical of the paraffin used in the experiment**

| Physicochemical characteristic | Measurement method | Range of values |
|---|---|---|
| Melting point (°C) | ASTM D-127 | 58-60 |
| Kinematic viscosity 100°C (cSt)* | ASTM D-445 | 4 |
| Penetration (dmm) | ASTM D-1321 | <17 |
| Oil content (p%) | ASTM D-721 | <0.5 |
| Hydrocarbon distribution | Gas chromatography | C22-C50 |

| | | |
|---|---|---|
| *ASTM: American Society for Testing and Materials* *(cSt) centistokes* | | |

### Emulsion paraffin:

In order to study the effect of the paraffin emulsion, an anionic/non-ionic emulsion of the paraffin described above was formulated. The characteristics of this emulsion are specified in Table 5.

**Table 5. Physicochemical characteristics typical of the emulsion used in the experiment**

| Physicochemical characteristic | Measurement method | Range of values |
|---|---|---|
| Solid content (%) | Evaporation | 55 |
| Viscosity (cPs)* | Rotational viscosimeter | 200 |
| pH | Ag/AgCl electrode | 8.5 |
| Mechanical stability | Pumping | STABLE |

| | | |
|---|---|---|
| *(cPs) centipoises* | | |

### 2.2. MAXIMUM PERCENTAGES OF ESSENTIAL OILS

The microbiological assays were performed with active paraffin with the different natural essences: cinnamon, cinnamon II, oregano, clove, thyme and citronella.

The incorporation percentages of the different essential oils vary within the range that it admits, without the mechanical properties of paraffin being modified for each essence (see Table 6).

**Table 6. Maximum percentages of essential oils incorporated into the paraffin.**

| Essential oil | Percentage (by weight) |
|---|---|
| Cinnamon | 10% |
| Cinnamon II | 10% |
| Oregano | 6% |
| Clove | 6% |
| Citronella | 8% |
| Thyme | 6% |

### Example 3

### FORMULATION OF THE ACTIVE PACKAGING

The formulation of the active packaging was performed using the following methodology:
In the case of solid paraffin, the paraffin was melted at a temperature of 110 ± 5°C, adding the pertinent additives in each case and stirring for 25 ± 3 minutes; thereafter, the temperature was decreased to 95 ± 5°C and the essential oils were added, stirring again for 10 ± 1 minutes. Subsequently, the other additives were added and it was again stirred for 15 ± 1 min. It was allowed to cool to ambient temperature.

In the case of emulsion paraffin, the paraffin phase was prepared in the same manner as for solid paraffin, that is, the paraffin was melted at a temperature of 110 ± °C, adding the additives in the specified order and quantities, and stirring for 25 ± 3 minutes; thereafter, the temperature was decreased to 95 ± 5°C and the essential oils were added, stirring again for 10 ± 1 minutes. Subsequently, the other additives were added and it was again stirred for 15 ± 1 min.

At this point, contrary to the former case, the surfactants were added and it was left to cool to 70 ± 5°C. In a separate container water was heated to 70 ± °C and the water-soluble surfactants were added. The paraffin phase and the aqueous phase were made to react in a separate reactor at 70 ± 5°C. Subsequently, it was passed through a high-pressure homogeniser in order to adjust the particle size and, finally, it was passed through a heat exchanger in order to leave the emulsion at ambient temperature.

### Example 4

### ANTI-MICROBIAL ASSAY

For the anti-microbial assay: the ideal culture medium for the growth of each microorganism is added in a Petri dish. Culture media are the nutritive solutions that are used in the laboratory to culture microorganisms under the most favourable conditions. Subsequently, 100 µl of a dilution of the microorganism in question are inoculated (inoculation concentration 10⁴ CFU¹/ml) and spread throughout the culture medium using a sterile handle. Once the medium is inoculated, the active paraffined paper is placed on the surface opposite to the dish's inoculated medium and fastened with a flange. At all times, an effort is made so that the active paper is not in contact with the inoculated medium (see Figures 2a and 2b).

The Petri dish, thus arranged, is incubated in an oven under the conditions (see Table 7) that favour microbial growth. In all cases, a control assay is always performed, which follows an identical procedure to that described above, except that, instead of the active paper, the Petri dish lid is used.

**Table 7. Microbial culture conditions**

| Microorganism | Time (days) | Temperature (°C) |
|---|---|---|
| Gram+ | 2 | 30 |
| Gram- | 1 | 30 |
| Mildews | 7 | 36.5 |
| Yeasts | 2 | 30 |

| | | |
|---|---|---|
| Inoculation concentration: 10⁴ CFU¹/ml | | |

In order to estimate the microbial growth produced in the dishes, the viable cell count method is used. This count is performed by a direct count of the colony-forming units (CFU) that appear in the dish. The lower the number of CFU, the greater the anti-microbial activity of the active paper. Moreover, the number of CFU is compared to that of the control assay, which has not been in contact with the active paper.

The results obtained for these assays are listed below:
- In the case of the essential oils of oregano, clove, thyme and citronella, the paper coated with the active paraffin (at the maximum percentage of incorporation of the essence admitted by the paraffin) did not exhibit any anti-microbial activity whatsoever.
- In the case of the essential oil of cinnamon, the paraffin prepared in accordance with example 3 does have certain antifungal activity (it inhibits the growth of some species of fungi), but no antibacterial activity. Total inhibition of the P. *islandicum* mildew has been observed. The control dish (without the active paraffined paper) had a growth of 14 colony-forming units (CFU), whereas no CFU grew in the dish with paraffined paper with 4% cinnamon essence by weight.

In the case of the C. *albicans* yeast, the paraffin with 10% cinnamon led to total inhibition (0 CFU in the dish with the active paper versus ∼3,500 CFU in the control dish), but when the percentage of incorporation of cinnamon was decreased to 4%, no inhibition was observed; that is, the number of CFU is similar to that of the control dish, ∼3,500 CFU.

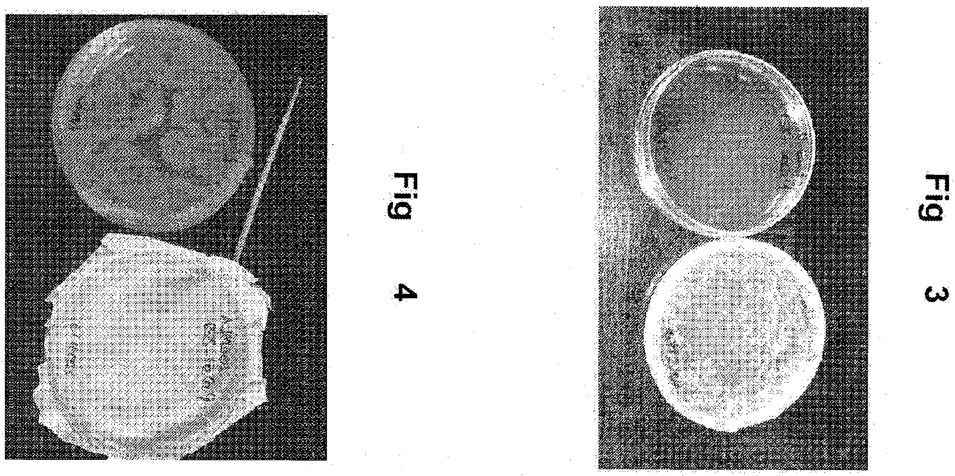

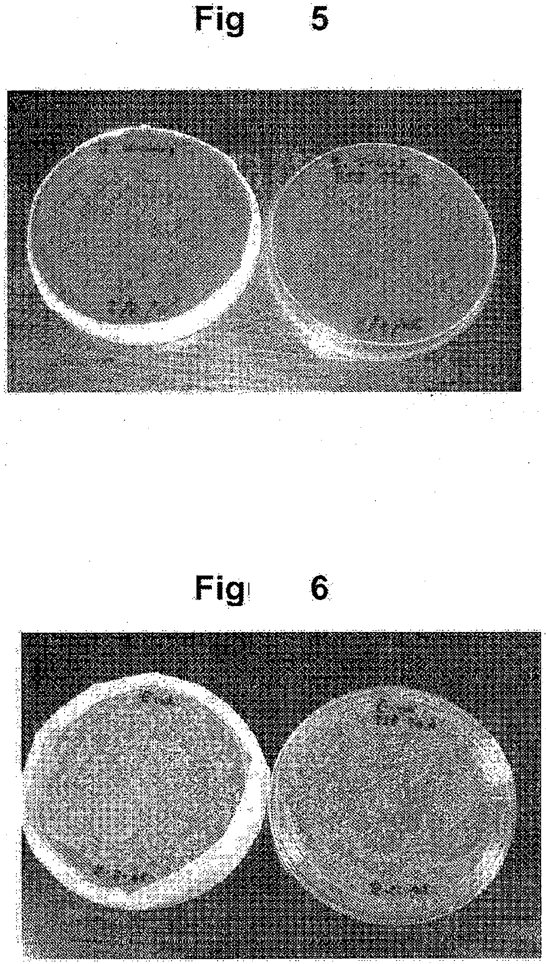

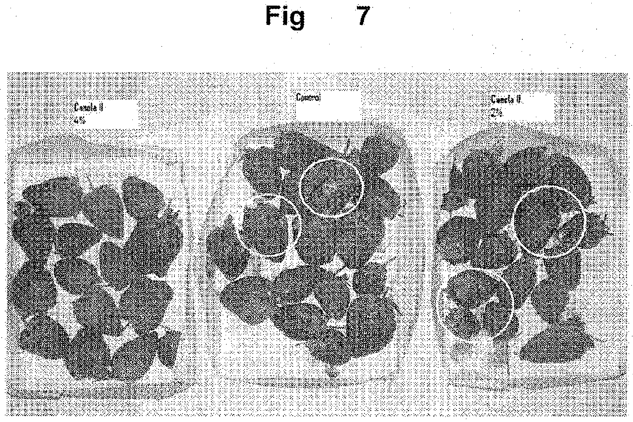

For bacteria, both Gram-positive and Gram-negative, the paraffined paper with essential oil of cinnamon does not inhibit the growth of the microorganisms. That is, there are no growth differences with respect to the control dishes.
- The best results were obtained for the essential oil of cinnamon II, that is, of all the essences assayed, the essential oil of cinnamon II is the one which showed the greatest anti-microbial activity.
   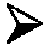 Assays with cinnamon II essence and Yeasts.
      Total inhibition of the C. *albicans* yeast was observed, with 4% essence by weight with respect to the paraffin. In Figure 3 it is compared to the control dish (right) and one can observe that total inhibition has taken place.
   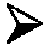 Assays with cinnamon II essence and Mildews.
      Total inhibition of all the mildews (A. *flavus,* P. *islandicum,* P. *roqueforti,* P. *nalgiovense* and E. *repens)* with a 4% essence percentage in the paraffin. In Figure 4 it is compared to the control dish (left) and one can observe that total inhibition has taken place.
   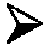 Assays with cinnamon II essence and Bacteria.
      Gram-positive bacteria (E. *faecalis,* L. *monocytogenes,* B. *cereus* and S. *aureus*). There is no inhibition at any essence concentration. See Figure 5, B. *cereus* with 4% cinnamon II (right) and control dish.
      Gram-negative bacteria (Y. *enterocolifica,* E. *coli,* S. *choleraesuis* and P. *aeruginosa*). There is a clear inhibitory effect, but one that is not very reproducible. For example, for the same percentage of essence in the paraffin and for the same microorganism, inhibition percentages (ratio of CFU in the assay dish with respect to the control) in a range between 100% and 0% were observed.

In many cases, there is not total inhibition, but it is clearly observed that the colony-forming units are smaller than the CFU in the control dish. (See Figure 6, E. *coli* with 6% cinnamon II [right] and control dish. A smaller size of the CFU is observed with respect to those of the control dish). Likewise, the colonies of P. *aeruginosa* in the dishes with the active packaging have a paler, more yellowish colour than the colonies in the control dish (bright green colour). In both cases, these characteristics indicate anomalies in the growth of the microorganism.

### Example 5

### STUDY OF ANTI-MICROBIAL ACTIVITY OVER TIME

In order to measure the duration of the anti-microbial activity of the active paper, assays separated in time were performed with sheets made on a given day and subsequently stored until the day of the assay. Two replicas were made for each assay, with the corresponding control dish. The active paper used has the following composition: office paper coated, in the usual manner, with emulsion paraffin (P-58) containing 4% cinnamon II by weight. The grammage applied is 4 g/m². These active paper sheets are stored in plastic (non-hermetic) folders at an approximate ambient temperature of between 3°C and 10°C. The microorganisms selected were the A. *flavus* mildew and the C. *albicans* yeast, since it is known from previous assays that, at this percentage of cinnamon II (4%), they totally inhibit colony growth. Gram-negative bacteria were not studied since previous results show that the inhibition is partial and not very reproducible.

### Results:

Both for C. *albicans* and A. *flavus,* total inhibition was observed for both replicas with a sheet storage time of up to 71 days. In the case of the mildew, the controls range between 104 and 105 colony-forming units. In the yeast, the controls showed between 300 and 900 CFU.

### Example 6

### RESULTS OF THE ASSAY OF THE ACTIVE PACKAGING FOR THE PRESERVATION OF STRAWBERRIES

In this example, different varieties of strawberries were used as the perishable foods.

The time results of the strawberries in contact with a control packaging with paraffined paper WITHOUT incorporated essence were compared to those using paraffined paper with cinnamon II essence incorporated at 4% by weight.

6 days after the beginning of the assay, it was observed that the strawberries in the packaging with 4% cinnamon II do not exhibit appreciable alterations even in the initial stage, as shown by the absence of juice exudation. On the other hand, the control packaging shows, together with exudation, proliferation of mildew.

## Claims

1. Active packaging for foods capable of inhibiting the growth of food pathogens, which comprises paraffin and cinnamon essential oil fortified and additived with cinnamaldehyde, wherein the fortified cinnamon is incorporated into the paraffin.

2. Active packaging according to claim 1, where the essential oil is present in percentage between 0.5% and 10% of the total weight.

3. Active packaging according to either of claims 1 and 2, where the cinnamon essential oil is present in percentage between 1% and 10% of the total weight.

4. Active packaging according to any of claims 1 to 3, where the cinnamon essential oil is present in percentage between 1% and 6% of the total weight.

5. Active packaging according to claim 4, where the cinnamon essential oil is present in percentage between 2% and 6% of the total weight.

6. Active packaging according to any of claims 1 to 5, where the paraffin formulation is an emulsion.

7. Active packaging according to any of claims 1 to 6, where the paraffin formulation comprises paraffin with CAS number: 64742-51-4.

8. Active packaging according to any of claims 1 to 7, where the paraffin formulation incorporates additives.

9. Active packaging according to any of claims 1 to 8, where the paraffin formulation incorporates a surfactant in its composition.

10. Active packaging according to claim 1, where the support may be made of paper, cardboard, wood, cork or aluminium.

11. Active packaging according to claim 1, where the grammage of the active coating is between 1 and 15 g/m².

12. Active packaging according to any of claims 1 to 11, where the food pathogens which it is capable of inhibiting are Gram-negative bacteria (Escherichia *coli,* Pseudomonas *aeruginosa,* Yersinia *enterocolitica* or Salmonella *choleraesuis*).

13. Active packaging according to any of claims 1 to 11, where the food pathogens which it is capable of inhibiting are Gram-positive bacteria (Staphylococcus *aureus,* Lysteria *monocytogenes*, Enterococcus *faecalis* or Bacillus *cereus).*

14. Active packaging according to any of claims 1 to 11, where the food pathogens which it is capable of inhibiting are mildews (Penicillium *islandicum,* Penicillium *roqueforti,* Penicillium *nalgiovense,* Aspergillus *flavus* or Eurotium *repens).*

15. Active packaging according to any of claims 1 to 11, where the food pathogens which it is capable of inhibiting are C. *albicans* yeasts.

16. Use of the active packaging according to any of claims 1 to 15, for the inhibition of the growth of food pathogens.

17. Use of the active packaging according to any of claims 1 to 15, as an antioxidant, preservative, anti-microbial or antifungal agent for any type of foods, dry or moist.

18. Use of the active packaging according to any of claims 1 to 15, to inhibit the growth of Gram-negative bacteria (Escherichia *coli,* Pseudomonas *aeruginosa,* Yersinia *enterocolitica* or Salmonella *choleraesuis*), Gram-positive bacteria (Staphylococcus *aureus,* Lysteria *monocytogenes,* Enterococcus *faecalis* or Bacillus *cereus*), mildews (Penicillium *islandicum,* Penicillium *roqueforti,* Penicillium *nalgiovense,* Aspergillus *flavus* or Eurotium *repens*) or the C. *albicans* yeast.

19. Use of the active packaging according to any of claims 1 to 15, as a protector by generating an active atmosphere or by direct contact with the food.

20. Method of obtaining an active packaging according to claims 1 to 15, which comprises the following steps:
a) Preparation of the paraffin
b) Addition of the cinnamon essential oil fortified and additived with cinnamaldehyde
c) Incorporation of the formulation obtained in the previous steps into a material that acts as a support.

## Patentansprüche

1. Aktive Verpackung für Lebensmittel, die in der Lage ist, das Wachstum von Lebensmittelpathogenen zu hemmen, und die Paraffin und mit Zimtaldehyd angereichertes und versetztes ätherisches Zimtöl umfasst, wobei der angereicherte Zimt in das Paraffin eingearbeitet ist.

2. Aktive Verpackung nach Anspruch 1, wobei das ätherische Öl in einem Prozentsatz zwischen 0,5 % und 10 % in Bezug auf das Gesamtgewicht vorliegt.

3. Aktive Verpackung nach einem der Ansprüche 1 oder 2, wobei das ätherische Zimtöl in einem Prozentsatz zwischen 1 % und 10 % in Bezug auf das Gesamtgewicht vorliegt.

4. Aktive Verpackung nach einem der Ansprüche 1 bis 3, wobei das ätherische Zimtöl in einem Prozentsatz zwischen 1 % und 6 % in Bezug auf das Gesamtgewicht vorliegt.

5. Aktive Verpackung nach Anspruch 4, wobei das ätherische Zimtöl in einem Prozentsatz zwischen 2 % und 6 % in Bezug auf das Gesamtgewicht vorliegt.

6. Aktive Verpackung nach einem der Ansprüche 1 bis 5, wobei die Paraffinformulierung eine Emulsion ist.

7. Aktive Verpackung nach einem der Ansprüche 1 bis 6, wobei die Paraffinformulierung Paraffin mit der CAS-Nummer 64742-51-4 umfasst.

8. Aktive Verpackung nach einem der Ansprüche 1 bis 7, wobei die Paraffinformulierung Zusatzstoffe enthält.

9. Aktive Verpackung nach einem der Ansprüche 1 bis 8, wobei die Paraffinformulierung eine oberflächenaktive Substanz in ihrer Zusammensetzung enthält.

10. Aktive Verpackung nach Anspruch 1, wobei der Träger aus Papier, Pappe, Holz, Kork oder Aluminium hergestellt sein kann.

11. Aktive Verpackung nach Anspruch 1, wobei das Grammgewicht der aktiven Beschichtung zwischen 1 und 15 g/m² liegt.

12. Aktive Verpackung nach einem der Ansprüche 1 bis 11, wobei die Lebensmittelpathogene, zu deren Hemmung sie in der Lage ist, Gram-negative Bakterien (*Escherichia coli, Pseudomonas aeruginosa, Yersinia enterocolitica* oder *Salmonella choleraesius*) sind.

13. Aktive Verpackung nach einem der Ansprüche 1 bis 11, wobei die Lebensmittelpathogene, zu deren Hemmung sie in der Lage ist, Gram-positive Bakterien (*Staphylococcus aureus, Lysteria monocytogenes, Enterococcus faecalis* oder *Bacillus cereus*) sind.

14. Aktive Verpackung nach einem der Ansprüche 1 bis 11, wobei die Lebensmittelpathogene, zu deren Hemmung sie in der Lage ist, Schimmelpilze *(Penicillium islandicum, Penicillium roqueforti, Penicillium nalgiovense, Aspergillus flavus* oder *Eurotium repens*) sind.

15. Aktive Verpackung nach einem der Ansprüche 1 bis 11, wobei die Lebensmittelpathogene, zu deren Hemmung sie in der Lage ist, die Hefen C. *albicans* sind.

16. Verwendung der aktiven Verpackung nach einem der Ansprüche 1 bis 15 zur Hemmung des Wachstums von Lebensmittelpathogenen.

17. Verwendung der aktiven Verpackung nach einem der Ansprüche 1 bis 15 als Antioxidans, Konservierungsstoff, Antimikrobenmittel oder Antimykotikum für jegliche Art von trockenen oder feuchten Lebensmitteln.

18. Verwendung der aktiven Verpackung nach einem der Ansprüche 1 bis 15 zur Hemmung des Wachstums von Gram-negativen Bakterien (*Escherichia coli, Pseudomonas aeruginosa, Yersinia enterocolitica* oder *Salmonella choleraesius*), Gram-positiven Bakterien (*Staphylococcus aureus, Lysteria monocytogenes, Enterococcus faecalis* oder *Bacillus cereus*), Schimmelpilzen (*Penicillium islandicum, Penicillium roqueforti, Penicillium nalgiovense, Aspergillus flavus* oder *Eurotium repens*) oder der Hefe C. *albicans.*

19. Verwendung der aktiven Verpackung nach einem der Ansprüche 1 bis 15 als Schutzmittel durch Erzeugung einer aktiven Atmosphäre oder durch direkten Kontakt mit dem Lebensmittel.

20. Verfahren zur Herstellung einer aktiven Verpackung nach einem der Ansprüche 1 bis 15, das folgende Schritte umfasst:
a) Vorbereitung des Paraffins
b) Zugabe des mit Zimtaldehyd angereicherten und versetzen ätherischen Zimtöls
c) Einarbeitung der in den vorhergehenden Schritten erhaltenen Formulierung in ein Material, das als Träger dient.

## Revendications

1. Emballage actif pour aliments capable d'inhiber la croissance de pathogènes alimentaires, celui-ci comprend de la paraffine et de l'huile essentielle de cannelle enrichie et renforcée par l'addition de cinnamaldéhyde, où la cannelle enrichie est incorporée dans la paraffine.

2. Emballage actif selon la revendication 1, où l'huile essentielle est présente dans un pourcentage compris entre 0.5% et 10% du poids total.

3. Emballage actif selon l'une quelconque des revendications 1 ou 2, où l'huile essentielle de cannelle est présente dans un pourcentage compris entre 1% et 10% du poids total.

4. Emballage actif selon l'une quelconque des revendications 1 à 3, où l'huile essentielle de cannelle est présente dans un pourcentage compris entre 1% et 6% du poids total.

5. Emballage actif selon la revendication 4, où l'huile essentielle de cannelle est présente dans un pourcentage compris entre 2% et 6% du poids total.

6. Emballage actif selon l'une quelconque des revendications 1 à 5, où la formulation de paraffine est une émulsion.

7. Emballage actif selon l'une quelconque des revendications 1 à 6, où la formulation de paraffine comprend de la paraffine dont le numéro CAS est : 64742-51-4.

8. Emballage actif selon l'une quelconque des revendications 1 à 7, où la formulation de paraffine contient des additifs.

9. Emballage actif selon l'une quelconque des revendications 1 à 8, où la formulation de paraffine contient un tensioactif dans sa composition.

10. Emballage actif selon la revendication 1, où le support peut être du papier, du carton, du bois, du liège ou de l'aluminium.

11. Emballage actif selon la revendication 1, où le grammage du revêtement actif est compris entre 1 et 15 g/m².

12. Emballage actif selon l'une quelconque des revendications 1 à 11, où les pathogènes alimentaires que ce dernier peut inhiber sont des bactéries gram-négatives (Escherichia *coli,* Pseudomonas *aeruginosa,* Yersinia *enterocolitica* ou Salmonella *choleraesuis*).

13. Emballage actif selon l'une quelconque des revendications 1 à 11, où les pathogènes alimentaires que ce dernier peut inhiber sont des bactéries gram-positives (Staphylococcus *aureus,* Lysteria *monocytogenes,* Enterococcus *faecalis* ou Bacillus *cereus*).

14. Emballage actif selon l'une quelconque des revendications 1 à 11, où les pathogènes alimentaires que ce dernier peut inhiber sont des moisissures (Penicillium *islandicum,* Penicillium *roqueforti,* Penicillium *nalgiovense,* Aspergillus *flavus* ou Eurotium *repens*).

15. Emballage actif selon l'une quelconque des revendications 1 à 11, où les pathogènes alimentaires que ce dernier peut inhiber sont des levures de l'espèce Candida *albicans.*

16. Utilisation de l'emballage actif selon l'une quelconque des revendications 1 à 15, pour inhiber la croissance des pathogènes alimentaires.

17. Utilisation de l'emballage actif selon l'une quelconque des revendications 1 à 15, en tant qu'antioxydant, conservateur, agent antimicrobien ou antifongique pour tout type d'aliments, secs ou humides.

18. Utilisation de l'emballage actif selon l'une quelconque des revendications 1 à 15, pour inhiber la croissance des bactéries gram-négatives (Escherichia *coli,* Pseudomonas *aeruginosa,* Yersinia *enterocolitica* ou Salmonella *choleraesuis*), des bactéries gram-positives (Staphylococcus *aureus,* Lysteria *monocytogenes,* Enterococcus *faecalis* ou Bacillus *cereus*), des moisissures (Penicillium *islandicum,* Penicillium *roqueforti,* Penicillium *nalgiovense,* Aspergillus *flavus* ou Eurotium *repens*) ou des levures de l'espèce Candida *albicans.*

19. Utilisation de l'emballage actif selon l'une quelconque des revendications 1 à 15, en tant que protecteur au moyen d'une atmosphère active ou en contact direct avec les aliments.

20. Méthode d'obtention d'un emballage actif selon les revendications 1 à 15, comprenant les étapes suivantes :
a) Préparation de la paraffine
b) Addition de l'huile essentielle de cannelle enrichie et renforcée avec le cinnamaldéhyde
c) Incorporation de la formulation obtenue dans les étapes précédentes dans une substance qui sert de support.
